# EUROPEAN PATENT SPECIFICATION

(11) **EP 2 538 900 B1**
(45) Date of publication and mention of the grant of the patent: **13.12.2017**
(21) Application number: 11706952.6
(22) Date of filing: 28.02.2011
(51) Int. Cl.: A61F 9/007

(54) **AN OPHTHALMIC SYSTEM AND A COMPUTER PROGRAM PRODUCT**
AUGENÄRZTLICHES SYSTEM UND COMPUTERPROGRAMMPRODUKT
SYSTÈME OPHTALMIQUE, PROCÉDÉ ET PRODUIT PROGRAMME D'ORDINATEUR

(30) Priority: 26.02.2010 NL 2004308
(43) Date of publication of application: 02.01.2013
(73) Proprietor: D.O.R.C. Dutch Ophthalmic Research Center (International) B.V., 3214 VN Zuidland (NL)
(72) Inventor: STALMANS, Peter, 3000 Leuven (BE); VIJFVINKEL, Gerrit Jan, 3211 BA Geervliet (NL)
(74) Representative: V.O.
(86) International application number: PCT/NL2011/050139
(87) International publication number: WO 2011/105909

(56) References cited:
- EP-A1- 1 647 248
- WO-A2-2007/001929
- DE-U1- 29 610 419
- US-A- 4 841 984
- US-A1- 2004 097 870

## Description

The invention relates to an ophthalmic system for controlling an infusion liquid pressure at a distal end of an ophthalmic irrigation device penetrating an eye and being connected to an irrigation line for feeding the device with an infusion liquid, comprising a processor that is arranged for estimating a compensation pressure for compensating an internal pressure loss in the eye due to surgical acts in the eye, for dynamically adjusting, in response to the estimated compensation pressure, the pressure of the irrigation liquid in the irrigation line.

In ophthalmic surgery, small probes are inserted into an eye to cut, remove or otherwise manipulate tissue. Typically, the interior of the eye is flushed with an infusion liquid by flowing the liquid into the eye via an ophthalmic irrigation device penetrating the eye. The irrigation device is fed by an irrigation line that is pressurized. During manipulating tissue in the interior of the eye, an amount of fluid leaving the eye via the insert opening can vary over time, e.g. depending on the surgical acts.

In prior art systems, such a fluid flow leaving the eye is sensed. The sensor information is fed back to a controlling system controlling the pressure of the infusion liquid at the distal end of the irrigation device.

The aim of the present invention is to provide an alternative of using a fluid flow measurement for the estimation of the compensation pressure. Thereto, according to the invention, the processor is arranged for performing the estimation step based on a configuration and/or actual operation of an ophthalmic surgical device performing the surgical acts in the eye.

By estimating the compensation pressure for compensating an internal pressure loss in the eye due to surgical acts in the eye, based on a configuration and/or actual operation of an ophthalmic surgical device performing the surgical acts, a compensation pressure can be found without the use of additional sensors, thereby simplifying the controlling system and making the system more robust. Further, since data concerning the surgical device configuration and actual operation can be obtained in a relatively great detail, the estimation of the compensation pressure can be more accurate. In addition, the step of dynamically adjusting the pressure of the irrigation liquid in the irrigation line can be performed faster, since the information of a changing operation state of the surgical device can be available earlier, e.g. by retrieving electrical operation instructions of the surgical device, thereby improving the dynamic behavior of the pressure compensation, so that the internal pressure in the eye can be kept in a smaller pressure range.

By using the system according to the invention, which is defined by the features of claim 1, the intraocular eye pressure can be kept stable at a value set by the surgeon, regardless of the surgical procedure.

It is noted that publication US 4 841 984 discloses an ophthalmic device and system for measuring and controlling a relative fluid pressure inside an ocular globe, using a pressure transducer in direct fluid communication with the fluid in the globe.

Further, the invention relates to a computer program product. A computer program product may comprise a set of computer executable instructions stored on a data carrier, such as a CD or a DVD. The set of computer executable instructions, which allow a programmable computer to carry out the method as defined above, may also be available for downloading from a remote server, for example via the Internet. The computer program product of the invention is defined by the features of claim 15. Further advantageous embodiments according to the invention are described in the following claims.

By way of example only, embodiments of the present invention will now be described with reference to the accompanying figures in which
Fig. 1 shows a schematic side view of a first embodiment of an ophthalmic surgical system according to the invention;
Fig. 2 shows a schematic side view of a second embodiment of an ophthalmic surgical system according to the invention; and
Fig. 3 shows a flow chart of an embodiment of using the invention.

It is noted that the figures show merely a preferred embodiment according to the invention. In the figures, the same reference numbers refer to equal or corresponding parts.

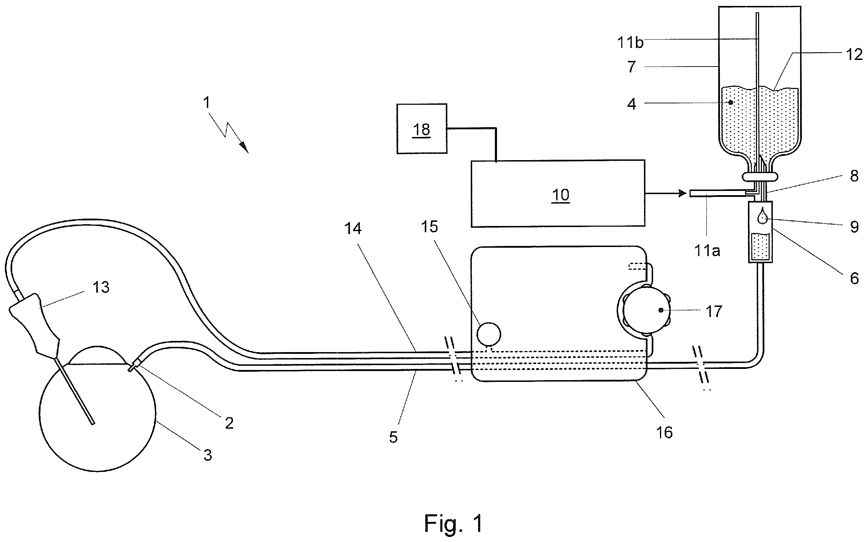

Figure 1 shows a schematic side view of a first embodiment of an ophthalmic surgical system 1 according to the invention. The system 1 comprises an irrigation canule 2 that penetrates the eye 3 for providing an irrigation liquid 4 into the interior of the eye. The system 1 further comprises an irrigation line 5 having a first end connected with the irrigation canule 2 and a second end connected with a drip chamber 6 that forms also part of the ophalmic surgical system 1. The system 1 further comprises an infusion bottle 7 that is partly filled with the irrigation liquid 4. The bottle 7 is further filled with gas for feeding and pressurizing the irrigation line. The bottle 7 includes a spike 8 for feeding the infusion liquid 4 to the drip chamber 6 with irrigation liquid droplets 9 via the chamber 6 entry. The liquid in the drip chamber 6 is in fluid communication, via its exit port and the irrigation line 5 also called infusion line, with the liquid in the irrigation canule 2. The system 1 also comprises a gas pressurizing unit 10 that is connected via a gas line 11a,b provided on the bottle 7 to the upper internal part of the bottle for dynamically adjusting the pressure of the gas in the infusion bottle above the infusion liquid surface 12.

The system 1 further comprises an ophthalmic surgical device implemented as a vitreous cutter 13 penetrating the eye at a separate location. The vitreous cutter 13 is connected, via an aspiration line 14 to an aspiration pump 15 for providing an under pressure to an exit port of the vitreous cutter 13. The aspiration line 14 and the aspiration pump 15 form an aspiration device. Any fluid and other material such as cut eye tissue that is aspirated, is collected in a cartridge 16. The aspiration pump 15 is driven by an electrical motor 17.

During operation of the system 1 an irrigation liquid pressure is exerted, at the distal end of the irrigation canule 2, on the liquid in the interior of the eye 3. The pressure is composed of a static part and a time varying, dynamic part. The time varying component is interrelated to actual surgical acts in the eye as will be explained below. The static component of the estimated pressure compensation is realized as a hydrostatic pressure by placing the infusion bottle 7 at a greater height than the vertical level of the treated eye 3, and/or a preset pressure of gas exerting a force on the infusion liquid surface 12 in the infusion bottle 7.

In order to apply a pressure that corresponds with a pressure variation in the eye, an estimation of a compensation pressure is computed for compensating an internal pressure loss in the eye due to surgical acts in the eye 3. Thereto, the system 1 comprises a processor 18 controlling the gas pressurizing unit 10. The estimation is based on a configuration and/or actual operation of the vitreous cutter 13 performing the surgical acts in the eye 3. As a consequence, the pressure of the irrigation liquid in the irrigation line 5 can dynamically and accurately be adjusted in response to the estimated compensation pressure.

In estimating the compensation pressure, the geometry and dimensions of the ophthalmic surgical device 13 is taken into account. As an example, the constellation of the vitreous cutter type forms a basis for the computation of the compensation. More specifically, the internal diameter of the cutter is taken into account, as the aspiration flow is directly dependent on the internal diameter of the cutter 13.

The actual operation of the ophthalmic surgical device 13 includes a cutting rate of the vitreous cutter 13. It appears that the aspiration flow is higher when the cutter is at rest than in a situation when the cutter is cutting. Generally, the aspiration flow decreases when the cutting rate increases.

Additionally, the estimation step can be based on an internal diameter of the irrigation canule 2, since the irrigation flow, and therefore the accumulation in eye pressure, is highly dependent on the internal diameter of the canule 2.

Further, aspiration characteristics of the aspiration device, such as an actual aspiration vacuum and/or aspiration flow, can optionally serve as a basis for estimating a compensation pressure.

The processor 18 controls the gas pressurizing unit 10 such that the pressure of the gas in the infusion bottle 7 is dynamically adjusted in response to the estimated compensation pressure. Preferably, the pressure compensation is computed periodically, so that a set intraocular eye pressure can be maintained. The computation period is preferably chosen relatively small, e.g. smaller than 1 second or smaller than 0.1 second.

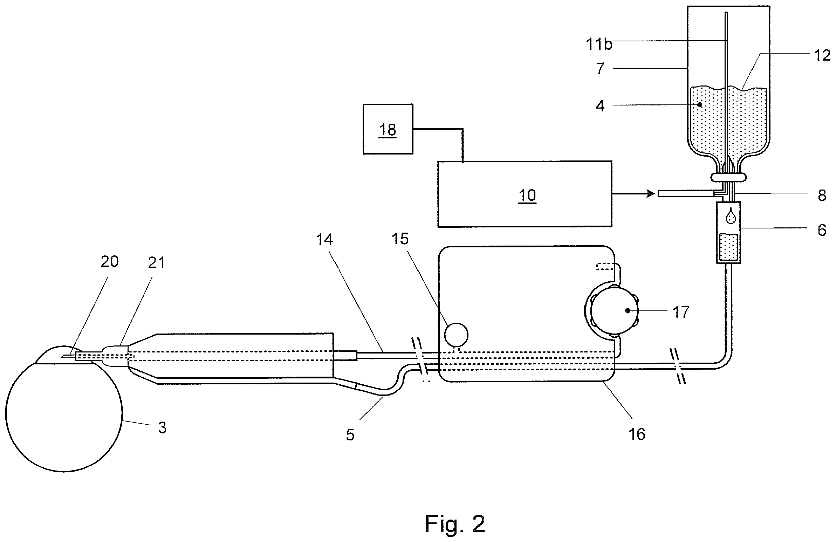

Figure 2 shows a schematic side view of a second embodiment of an ophthalmic surgical system 1 according to the invention. The system 1 now includes an ophthalmic surgical device that has been implemented as a phaco needle 20. Further, the ophthalmic irrigation device is now implemented as a sleeve 21 surrounding the needle 20. As such, the ophthalmic surgical device and the ophthalmic irrigation device have been integrated in a single device, viz. a phaco device for performing phaco emulsification procedures in the anterior chamber of the eye 3. In estimating the compensation pressure, the constellation of the phaco needle type can be taken into account, more specifically the internal diameter of the phaco needle 20. Further, as an alternative or additionally, the geometry of the sleeve 21 around the phaco needle 20 can be taken into account.

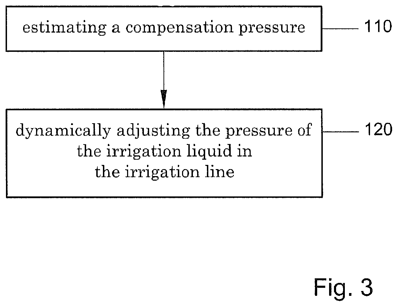

Figure 3 shows a flow chart of a method of using the invention. Such method is used for controlling an infusion liquid pressure at a distal end of an ophthalmic irrigation device penetrating an eye. The method comprises a step of estimating 110 a compensation pressure for compensating an internal pressure loss in the eye due to surgical acts in the eye; and a step of dynamically adjusting 120 the pressure of irrigation liquid in an irrigation line connected to the irrigation device for feeding the device with an infusion liquid, in response to the estimated compensation pressure. The estimation step 110 is based on a configuration and actual operation of a surgical device performing the surgical acts in the eye.

Preferably, the estimation step 110 is directly based on data concerning configuration and/or actual operation of the ophthalmic surgical device, so that the use of a sensor measuring a pressure or fluid flow is superfluous.

By estimating a compensation pressure that is equal to the internal pressure loss in the eye due to surgical acts in the eye, variations in a fluid flow leaving the eye can be compensated, thereby maintaining a mainly constant pressure in the eye.

The method of controlling an infusion liquid pressure at a distal end of an ophthalmic irrigation device penetrating an eye can be performed using dedicated hardware structures, such as FPGA and/or ASIC components. Otherwise, the method can also at least partially be performed using a computer program product comprising instructions for causing a processor of the computer system to perform the above described steps of the method according to the invention. All steps can in principle be performed on a single processor. However it is noted that at least one step can be performed on a separate processor, e.g. the step of estimating.

The invention is not restricted to the embodiments described herein. It will be understood that many variants are possible.

The gas that is applied for exerting a pressure on the infusion liquid surface 12 is e.g. air. However, in principle, also other gas types can be used.

It is noted that the pressure on the infusion liquid in the infusion line and at the distal end of the irrigation device can in principle be controlled in another way, e.g. by exerting a force on the liquid via a movable membrane that contacts the liquid, or by using a valve pressurizing system.

It is further noted that the ophthalmic system can be composed of several modules, e.g. a module including the surgical device, a module including the irrigation device with the irrigation line and infusion bottle, and a module including the controller 18 for controlling the gas pressurizing unit 10.

These and other embodiments will be apparent for the person skilled in the art and are considered to lie within the scope of the invention as defined in the following claims.

## Claims

1. An ophthalmic system (1) for controlling an infusion liquid pressure at a distal end of an ophthalmic irrigation device (13, 20) penetrating an eye (3) and being connected to an irrigation line (5) for feeding the device with an infusion liquid (4), wherein the system comprises a processor (18), **characterized in that** the processor is arranged for estimating a compensation pressure for compensating an internal pressure loss in the eye due to surgical acts in the eye, for dynamically adjusting, in response to the estimated compensation pressure, the pressure of the irrigation liquid in the irrigation line, wherein the estimation step is based on a configuration and/or actual operation of an ophthalmic surgical device performing the surgical acts in the eye and wherein, in the estimation step, the geometry and dimensions of the ophthalmic surgical device are taken into account.

2. An ophthalmic system according to claim 1, wherein the estimation step is directly based on data concerning configuration and/or actual operation of the ophthalmic surgical device.

3. An ophthalmic system according to claim 1 or 2, wherein the compensation pressure to be estimated is the internal pressure loss in the eye due to surgical acts in the eye.

4. An ophthalmic system according to any of the preceding claims, wherein the configuration of the ophthalmic surgical device includes the geometry and dimensions of the ophthalmic surgical device.

5. An ophthalmic system according to any of the preceding claims, wherein the configuration of the ophthalmic surgical device includes a constellation of a vitreous cutter type or a phaco needle type.

6. An ophthalmic system according to claim 5, wherein the constellation includes the internal diameter of the cutter and the needle, respectively, of the vitreous cutter and phaco needle.

7. An ophthalmic system according any of the preceding claims, wherein the configuration of the ophthalmic surgical device includes aspiration characteristics of an aspiration device connected to the ophthalmic surgical device.

8. An ophthalmic system according to any of the preceding claims, wherein the actual operation of the ophthalmic surgical device includes a cutting rate.

9. An ophthalmic system according to any of the preceding claims, wherein the estimation step is further based on an internal diameter of the ophthalmic irrigation device implemented as an irrigation canule.

10. An ophthalmic system according to any of the preceding claims, wherein the estimation step is further based on the geometry of the ophthalmic irrigation device formed as a sleeve enclosing a phaco needle.

11. An ophthalmic system according to any of the preceding claims, further comprising an ophthalmic irrigation device for penetrating an eye and an irrigation line connected to the irrigation device for feeding the device with an infusion liquid.

12. An ophthalmic system according to claim 11, further comprising an infusion bottle filled with a gas and an infusion liquid for feeding and pressurizing the irrigation line, and further comprising a gas pressurizing unit that is arranged for dynamically adjusting the pressure of the gas in the infusion bottle in response to the estimated compensation pressure.

13. An ophthalmic system according to any of the preceding claims, wherein the ophthalmic surgical device and the ophthalmic irrigation device have been integrated in a single device, and wherein the static component of the estimated pressure compensation is realized as a hydrostatic pressure and/or a preset pressure of gas exerting a force on infusion liquid in the irrigation line.

14. An ophthalmic system according to any of the preceding claims, wherein the estimated compensation pressure is composed of a static component and a time varying component that is interrelated to actual surgical acts in the eye.

15. A computer program product for controlling an infusion liquid pressure at a distal end of an ophthalmic irrigation device (13, 20) penetrating an eye (3), **characterized in that** the computer program product comprises computer readable code for causing a processor (18) to perform the step of estimating a compensation pressure for compensating an internal pressure loss in the eye due to surgical acts in the eye, and dynamically adjusting the pressure of irrigation liquid (4) in an irrigation line (5) connected to the irrigation device for feeding the device with an infusion liquid, in response to the estimated compensation pressure, wherein the estimation step is based on a configuration and/or actual operation of a surgical device performing the surgical acts in the eye, and wherein, in the estimation step, the geometry and dimensions of the ophthalmic surgical device are taken into account.

## Patentansprüche

1. Augenärztliches System (1) zum Kontrollieren eines Infusionsflüssigkeitsdrucks an einem distalen Ende einer augenärztlichen Spülvorrichtung (13, 20), die ein Auge (3) penetriert und mit einer Spülleitung (5) verbunden ist, um die Vorrichtung mit einer Infusionsflüssigkeit (4) zu versorgen, wobei das System einen Prozessor (18) umfasst, **dadurch gekennzeichnet, dass** der Prozessor angeordnet ist zum Schätzen eines Ausgleichdrucks zum Ausgleichen eines inneren Druckverlustes in dem Auge aufgrund chirurgischer Handlungen in dem Auge, um, in Reaktion auf den geschätzten Ausgleichsdruck, den Druck der Spülflüssigkeit in der Spülleitung dynamisch anzupassen, wobei der Schätzungsschritt auf einer Konfiguration und/oder dem tatsächlichen Bedienen einer augenärztlichen chirurgischen Vorrichtung, welche die chirurgischen Handlungen in dem Auge vornimmt, basiert, und wobei in dem Schätzungsschritt die Geometrie und Abmessungen der augenärztlichen chirurgischen Vorrichtung berücksichtigt werden.

2. Augenärztliches System nach Anspruch 1, wobei der Schätzungsschritt direkt auf Daten betreffend die Konfiguration und/oder das tatsächliche Bedienen der augenärztlichen chirurgischen Vorrichtung basiert.

3. Augenärztliches System nach Anspruch 1 oder 2, wobei der zu schätzende Ausgleichsdruck der innere Druckverlust in dem Auge aufgrund chirurgischer Handlungen in dem Auge ist.

4. Augenärztliches System nach einem der vorhergehenden Ansprüche, wobei die Konfiguration der augenärztlichen chirurgischen Vorrichtung die Geometrie und Abmessungen der augenärztlichen chirurgischen Vorrichtung umfasst.

5. Augenärztliches System nach einem der vorhergehenden Ansprüche, wobei die Konfiguration der augenärztlichen chirurgischen Vorrichtung eine Konstellation eines glasartigen Schneidertyps oder eines Phaco-Nadeltpys umfasst.

6. Augenärztliches System nach Anspruch 5, wobei die Konstellation den Innendurchmesser des Schneiders und der Nadel von jeweils dem glasartigen Schneider und der Phaco-Nadel umfasst.

7. Augenärztliches System nach einem der vorhergehenden Ansprüche, wobei die Konfiguration der augenärztlichen chirurgischen Vorrichtung Ansaugmerkmale einer Ansaugvorrichtung, verbunden mit der augenärztlichen chirurgischen Vorrichtung, umfasst.

8. Augenärztliches System nach einem der vorhergehenden Ansprüche, wobei die tatsächliche Bedienung der augenärztlichen chirurgischen Vorrichtung eine Schneiderate umfasst.

9. Augenärztliches System nach einem der vorhergehenden Ansprüche, wobei der Schätzungsschritt ferner auf einem Innendurchmesser der augenärztlichen Spülvorrichtung, implementiert als eine Spülkanüle, basiert.

10. Augenärztliches System nach einem der vorhergehenden Ansprüche, wobei der Schätzungsschritt ferner auf der Geometrie der augenärztlichen Spülvorrichtung, geformt als ein Ärmel, der eine Phaco-Nadel umschließt, basiert.

11. Augenärztliches System nach einem der vorhergehenden Ansprüche, ferner umfassend eine augenärztliche Spülvorrichtung zum Penetrieren eines Auges und eine Spülleitung, verbunden mit der Spülvorrichtung, um die Vorrichtung mit einer Infusionsflüssigkeit zu versorgen.

12. Augenärztliches System nach Anspruch 11, ferner umfassend eine Infusionsflasche, gefüllt mit einem Gas und einer Infusionsflüssigkeit zur Versorgung und Druckbeaufschlagung der Spülleitung, und ferner umfassend eine Gasdruckbeaufschlagungseinheit, die angeordnet ist, um dynamisch den Druck des Gases in der Infusionsflasche als Reaktion auf den geschätzten Ausgleichsdruck anzupassen.

13. Augenärztliches System nach einem der vorhergehenden Ansprüche, wobei die augenärztliche chirurgische Vorrichtung und die augenärztliche Spülvorrichtung in eine einzige Vorrichtung integriert wurden, und wobei die statische Komponente des geschätzten Druckausgleichs als ein hydrostatischer Druck und/oder ein voreingestellter Druck von Gas, das eine Kraft auf die Infusionsflüssigkeit in der Spülleitung ausübt, realisiert ist.

14. Augenärztliches System nach einem der vorhergehenden Ansprüche, wobei der geschätzte Ausgleichsdruck aus einer statischen Komponente und einer zeitvariierenden Komponente, die in Wechselbeziehung mit tatsächlichen Handlungen im Auge ist, zusammengesetzt ist.

15. Computerprogrammprodukt zum Kontrollieren eines Infusionsflüssigkeitsdrucks an einem distalen Ende einer augenärztlichen Spülvorrichtung (13, 20), die ein Auge (3) penetriert, **dadurch gekennzeichnet, dass** das Computerprogrammprodukt computerlesbaren Code umfasst, um einen Prozessor (18) zu veranlassen, den Schritt der Schätzung eines Ausgleichsdrucks zum Ausgleichen eines Innendruckverlusts in dem Auge aufgrund chirurgischer Handlungen in dem Auge und zum dynamischen Anpassen des Drucks von Infusionsflüssigkeit (4) in einer Spülleitung (5), verbunden mit der Spülvorrichtung zum Versorgen der Vorrichtung mit einer Infusionsleitung als Reaktion auf den geschätzten Ausgleichsdruck auszuführen, wobei der Schätzungsschritt auf einer Konfiguration und/oder einer tatsächlichen Bedienung einer chirurgischen Vorrichtung, die chirurgische Handlungen in dem Auge vornimmt, basiert, und wobei in dem Schätzungsschritt die Geometrie und Abmessungen der augenärztlichen chirurgischen Vorrichtung berücksichtigt werden.

## Revendications

1. Un système ophtalmique (1) pour contrôler une pression de liquide de perfusion à une extrémité distale d'un dispositif d'irrigation ophtalmique (13, 20) pénétrant dans un oeil (3) et relié à une ligne d'irrigation (5) pour alimenter le dispositif avec un liquide de perfusion (4), lequel système comprend un processeur (18) **caractérisé en ce que** le processeur est conçu
- pour estimer une pression de compensation en vue de compenser une perte de pression interne dans l'oeil due à des actes chirurgicaux effectués sur l'oeil,
- pour ajuster de façon dynamique, en réponse à la pression de compensation estimée, la pression du liquide d'irrigation dans la ligne d'irrigation,
l'étape d'estimation étant basée sur une configuration et/ou un fonctionnement effectif d'un dispositif chirurgical ophtalmique effectuant des actes chirurgicaux sur l'oeil, et cette étape d'estimation prenant en considération la géométrie et les dimensions du dispositif chirurgical ophtalmique.

2. Un système ophtalmique selon la revendication 1, dans lequel l'étape d'estimation est directement basée sur des données concernant la configuration et/ou le fonctionnement réel du dispositif chirurgical ophtalmique.

3. Un système ophtalmique selon la revendication 1 ou 2, dans lequel la pression de compensation à estimer est la perte de pression interne dans l'oeil due à des actes chirurgicaux effectués sur l'oeil.

4. Un système ophtalmique selon l'une quelconque des revendications précédentes, dans lequel la configuration du dispositif chirurgical ophtalmique comprend la géométrie et les dimensions du dispositif chirurgical ophtalmique.

5. Un système ophtalmique selon l'une quelconque des revendications précédentes, dans lequel la configuration du dispositif chirurgical ophtalmique comprend une constellation d'un type de coupe vitreuse ou d'une aiguille de phaco-émulsification.

6. Un système ophtalmique selon la revendication 5, dans lequel la constellation comprend le diamètre interne du dispositif de coupe et l'aiguille, respectivement, du dispositif de coupe vitreuse et de l'aiguille de phaco-émulsification.

7. Un système ophtalmique selon l'une quelconque des revendications précédentes, dans lequel la configuration du dispositif chirurgical ophtalmique comprend des caractéristiques d'aspiration d'un dispositif d'aspiration relié au dispositif chirurgical ophtalmique.

8. Un système ophtalmique selon l'une quelconque des revendications précédentes, dans lequel le fonctionnement réel du dispositif chirurgical ophtalmique comprend une vitesse de coupe.

9. Un système ophtalmique selon l'une quelconque des revendications précédentes, dans lequel l'étape d'estimation est en outre basée sur un diamètre interne du dispositif d'irrigation ophtalmique mis en oeuvre en tant que canule d'irrigation.

10. Un système ophtalmique selon l'une quelconque des revendications précédentes, dans lequel l'étape d'estimation est en outre basée sur la géométrie du dispositif d'irrigation ophtalmique formé en tant que manchon enfermant une aiguille de phaco-émulsification.

11. Un système ophtalmique selon l'une quelconque des revendications précédentes, comprenant en outre un dispositif d'irrigation ophtalmique pour pénétrer dans un oeil et une ligne d'irrigation connectée au dispositif d'irrigation pour alimenter le dispositif avec un liquide de perfusion.

12. Un système ophtalmique selon la revendication 11, comprenant en outre une bouteille de perfusion remplie d'un gaz et d'un liquide de perfusion pour l'alimentation et la mise sous pression de la ligne d'irrigation et comprenant en outre une unité de pressurisation de gaz conçue pour ajuster de façon dynamique la pression du gaz dans la bouteille de perfusion en réponse à la pression de compensation estimée.

13. Un système ophtalmique selon l'une quelconque des revendications précédentes, dans lequel le dispositif chirurgical ophtalmique et le dispositif d'irrigation ophtalmique ont été intégrés dans un dispositif unique et dans lequel la composante statique de la compensation de pression estimée est réalisée sous la forme d'une pression hydrostatique et/ou d'une pression prédéfinie de gaz exerçant une force sur le liquide de perfusion dans la ligne d'irrigation.

14. Un système ophtalmique selon l'une quelconque des revendications précédentes, dans lequel la pression de compensation estimée est composée d'un composant statique et d'un composant variant dans le temps qui est en corrélation avec des actes chirurgicaux réels effectués sur l'oeil.

15. Produit formant programme d'ordinateur pour la régulation d'une pression de liquide de perfusion à une extrémité distale d'un dispositif d'irrigation ophtalmique (13, 20) pénétrant un oeil (3), **caractérisé en ce que** le produit formant programme d'ordinateur comprend un code lisible par ordinateur pour qu'un processeur (18) effectue les étapes consistant à :
- estimer une pression de compensation pour compenser une perte de pression interne dans l'oeil due à des actes chirurgicaux effectués sur l'oeil ; et
- ajuster de façon dynamique la pression du liquide d'irrigation (4) dans une ligne d'irrigation (5) reliée au dispositif d'irrigation pour alimenter le dispositif avec un liquide de perfusion, en réponse à la pression de compensation estimée, où l'étape d'estimation est basée sur une configuration et/ou une opération réelle d'un dispositif chirurgical réalisant les actes chirurgicaux sur l'oeil, et dans lequel cette étape d'estimation prend en considération la géométrie et les dimensions du dispositif chirurgical ophtalmique.
